# FASCICULE DE BREVET EUROPEEN

(11) **EP 3 928 699 B1**
(45) Date de publication et mention de la délivrance du brevet: **03.08.2022**
(21) Numéro de dépôt: 21181185.6
(22) Date de dépôt: 23.06.2021
(51) Int. Cl.: A61B 5/15, A61B 5/153, A61M 5/32, A61B 34/30

(54) **TÊTE DE PONCTION SANGUINE POUR MACHINE DE PRÉLÈVEMENT SANGUIN AUTOMATIQUE OU SEMI-AUTOMATIQUE**
BLUTENTNAHMEKOPF FÜR AUTOMATISCHES ODER HALBAUTOMATISCHES BLUTENTNAHMEGERÄT
BLOOD VENIPUNCTURE HEAD FOR AUTOMATIC OR SEMI-AUTOMATIC BLOOD SAMPLING MACHINE

(30) Priorité: 26.06.2020 FR 2006743
(43) Date de publication de la demande: 29.12.2021
(73) Titulaire: BHEALTHCARE, 44800 Saint-Herblain (FR)
(72) Inventeur: BRETEAU, Aliaume, 44321 NANTES CEDEX 3 (FR); DE CHAISEMARTIN, Jean-Baptiste, 44321 NANTES CEDEX 3 (FR); GEFFROY, Sylvain, 44321 NANTES CEDEX 3 (FR); RIVIERE, Alexandre, 44321 NANTES CEDEX 3 (FR)
(74) Mandataire: Bringer IP

(56) Documents cités:
- CN-A- 111 012 363
- US-A1- 2008 167 674
- US-A1- 2010 274 202
- US-A1- 2012 190 981
- US-A1- 2017 188 990

## Description

### Domaine technique de l'invention

L'invention concerne une tête de ponction sanguine destinée à équiper une machine de prélèvement sanguin automatique ou semi-automatique. L'invention concerne aussi une machine de prélèvement sanguin automatique ou semi-automatique équipée d'une tête de ponction sanguine selon l'invention.

### Arrière-plan technologique

Les établissements de santé réalisent chaque jour un très grand nombre de prises de sang. Ces opérations sont chronophages, répétitives et potentiellement dangereuses pour le personnel soignant et le patient compte tenu des risques de blessure qu'elles comportent liés par exemple à un tremblement du personnel soignant, sa fatigue, son inexpérience, un mauvais geste ou à un mauvais réflexe du patient lors de l'introduction de l'aiguille dans sa veine.

En outre, certains patients présentent des veines peu propices à une bonne prise de sang, ce qui peut nécessiter plusieurs tentatives de ponction du personnel soignant avant de pouvoir atteindre une veine permettant une prise de sang. Cette répétition des tentatives peut être douloureuse pour le patient et peut générer des blessures, ce qui ne fait que compliquer l'opération de prélèvement pour ces patients. US 2012/190981 divulgue un système d'insertion intraveineuse autonome comprend un bras de robot, un ou plusieurs capteurs fixés de manière pivotante au bras de robot pour recueillir des informations sur les sites d'insertion potentiels dans un bras de sujet, un dispositif médical fixé de manière pivotante au bras de robot, et un contrôleur en communication avec les capteurs et le bras de robot, dans lequel le contrôleur reçoit les informations des capteurs sur les sites d'insertion potentiels, et le contrôleur sélectionne un site d'insertion cible et dirige le bras de robot pour insérer le dispositif médical dans le site d'insertion cible.

De plus, la crise sanitaire mondiale de coronavirus appelle le développement de système limitant les contacts entre les patients et le personnel soignant et/ou pouvant assurer des dépistages massifs de la population.

Ainsi, le déposant a proposé dans le document WO2015158978 une machine de ponction automatique dans une veine d'un patient comprenant des moyens de capture d'image infrarouge du bras du patient, des moyens de détection d'une veine dans l'image capturée, un dispositif de maintien de la veine détectée, une aiguille et des moyens d'insertion de l'aiguille dans la veine détectée.

Un tel dispositif permet ainsi d'automatiser les opérations de prélèvement sanguin.

Cela étant, certaines opérations nécessaires au prélèvement sanguin, telles la pose d'un pansement, la gestion des tubes de prélèvement, le contrôle du prélèvement, etc., nécessitent toujours une présence humaine et/ou des équipements annexes.

Le déposant a donc cherché à améliorer le dispositif proposé et notamment à fournir une tête de ponction autonome destinée à équiper un ensemble mécatronique tel qu'un bras robotisé d'une machine de prélèvement sanguin automatique ou semi-automatique.

Dans tout le texte, on entend par machine automatique ou semi-automatique, une machine qui peut fonctionner sans une intervention humaine, à l'exception d'éventuelles opérations de chargement de la machine, de mise en route de la machine et/ou de contrôle de la machine.

### Objectifs de l'invention

L'invention vise à fournir une tête de ponction sanguine destinée à équiper un ensemble mécatronique tel qu'un bras robotisé d'une machine de ponction automatique ou semi-automatique.

L'invention vise en particulier à fournir une tête de ponction sanguine autonome qui puisse équiper tout type d'ensemble mécatronique, tel qu'un bras robotisé, de tout type de machine de prélèvement sanguin automatique ou semi-automatique.

L'invention vise aussi à fournir, dans au moins un mode de réalisation de l'invention, une tête de ponction sanguine qui permette d'embarquer une aiguille de prélèvement et qui permette d'assurer automatiquement la liaison fluidique entre l'aiguille de prélèvement et un tube de réception du sang prélevé.

L'invention vise aussi à fournir, dans au moins un mode de réalisation de l'invention, une tête de ponction sanguine qui puisse embarquer des tubes de prélèvement destinés à recueillir les prélèvements sanguins.

L'invention vise aussi à fournir, dans au moins un mode de réalisation de l'invention, une tête de ponction sanguine qui permette de vérifier la présence de sang dans les tubes de prélèvement.

L'invention vise aussi à fournir, dans au moins un mode de réalisation de l'invention, une tête de ponction sanguine qui permette de vérifier automatiquement le remplissage des tubes de prélèvements.

L'invention vise aussi à fournir, dans au moins un mode de réalisation de l'invention, une tête de ponction qui permet de changer automatiquement les tubes de prélèvement.

L'invention vise aussi à fournir, dans au moins un mode de réalisation de l'invention, une tête de ponction sanguine qui permette de positionner correctement le biseau de l'aiguille à l'opposé de la peau du membre du patient à ponctionner.

L'invention vise aussi à fournir, dans au moins un mode de réalisation de l'invention, une tête de ponction sanguine qui permette une pose automatique d'un pansement sur la peau du patient suite au prélèvement.

L'invention vise aussi à fournir, dans au moins un mode de réalisation de l'invention, une tête de ponction sanguine qui permette d'interrompre la ponction sanguine en cas d'anomalies détectées, et notamment le retrait de l'aiguille du membre du patient en cas de coupure de courant.

L'invention vise aussi à fournir, dans au moins un mode de réalisation de l'invention, une tête de ponction qui permet une désinfection automatique de la zone de ponction.

L'invention vise enfin à fournir une machine de prélèvement sanguin automatique ou semi-automatique équipée d'une tête de ponction selon l'invention.

### Exposé de l'invention

Pour ce faire, l'invention concerne une tête de ponction sanguine destinée à équiper un ensemble mécatronique tel qu'un bras robotisé d'une machine de prélèvement sanguin automatique ou semi-automatique configuré pour permettre le déplacement de ladite tête au-dessus d'un membre d'un patient à ponctionner.

La tête de ponction selon l'invention comprend :
- un châssis équipé de moyens de fixation amovible du châssis audit ensemble mécatronique,
- un porte-aiguille porté par ledit châssis et adapté pour recevoir une aiguille comprenant une portion biseautée destinée à percer la peau du membre du patient à ponctionner et une portion arrière destinée à permettre l'écoulement du sang prélevé vers un tube de prélèvement.

La tête de ponction selon l'invention est caractérisée en ce qu'elle comprend en outre :
- des moyens de déplacement linéaire dudit porte-aiguille configurés pour permettre, une fois ce dernier équipé d'une aiguille, d'armer la tête de ponction en vue d'insérer, sur commande dudit ensemble mécatronique, cette aiguille dans ledit membre dudit patient pour pouvoir effectuer un prélèvement sanguin,
- un dispositif de mise en liaison fluidique de ladite portion arrière de ladite aiguille montée sur ledit porte-aiguilles avec un tube de prélèvement destiné à recueillir le sang prélevé dans ledit membre du patient,
- un dispositif électromécanique de désarmement de la tête et de retrait d'urgence de ladite aiguille dudit membre du patient configuré pour permettre, sur commande, un retrait exclusivement mécanique de ladite aiguille de ponction dudit membre du patient.

Une tête de ponction selon l'invention embarque ainsi un porte-aiguille, des moyens de déplacement du porte-aiguille, un dispositif, par exemple motorisé, de mise en liaison fluidique de l'aiguille avec un tube de prélèvement (qui peut être embarqué sur la tête comme décrit ultérieurement ou agencé à distance de la tête et relié à l'aiguille par un moyen de connexion tel qu'un tube flexible) et un dispositif de désarmement et de retrait d'urgence, permettant ainsi d'assurer un prélèvement sanguin automatisé dans un membre d'un patient (qui peut être un membre supérieur du patient, aussi désigné par le terme bras d'un patient dans la suite ou un membre inférieur du patient). En d'autres termes, les différents éléments nécessaires au prélèvement sanguin du patient se déplacent avec la tête de ponction de sorte que ces éléments sont immédiatement accessibles par la tête, sans nécessiter un déplacement de l'ensemble mécatronique vers un équipement annexe ou l'intervention d'un opérateur. Une tête de ponction selon l'invention peut ainsi équiper n'importe quel bras robotisé pour former une machine de ponction automatique ou semi-automatique.

Dans tout le texte, les termes « bras robotisé » désignent un ensemble mécatronique configuré pour permettre le déplacement de la tête portée par cet ensemble mécatronique au-dessus du membre du patient à ponctionner. Ainsi, un tel bras robotisé peut être formé d'un arbre articulé, par exemple selon six-axes, et piloté par une commande numérique (prenant ainsi l'aspect d'un bras en tant que tel) ou de tout système automatisé équipé d'actionneurs (câbles, moteurs électriques, vérins, portants, glissières, etc.) permettant de déplacer, sur commande, une charge portée par le système (en l'occurrence la tête de ponction) vers le membre à ponctionner.

Dans tout le texte, le terme « aiguille » désigne une tige effilée comprenant une portion biseautée adaptée pour pouvoir percer la peau d'un patient et une portion arrière adaptée pour pouvoir être mise en communication de fluide avec un tube de prélèvement de sang ou tout consommable adapté pour assurer une collecte d'échantillons sanguins. Une telle aiguille au sens de l'invention peut être une aiguille de prélèvement en tant que tel, mais peut également être tout équipement pouvant être utilisé pour un prélèvement, sans néanmoins être initialement destiné à un tel prélèvement. Par exemple une aiguille au sens de l'invention peut être une aiguille épicrânienne, un cathéter, un micro perfuseur, etc. dont la fonction initiale n'est pas le prélèvement sanguin, mais qui peut néanmoins être utilisée pour un tel prélèvement. Dans tout le texte, les termes « aiguille de prélèvement » doivent être interprétés comme une aiguille au sens de l'invention.

Une tête de ponction selon l'invention comprend également un dispositif de mise en liaison fluidique de la portion arrière de l'aiguille montée sur le porte-aiguille avec un tube de prélèvement destiné à recueillir le sang prélevé dans ledit membre du patient.

Un tel dispositif de mise en liaison fluidique de la portion arrière de l'aiguille et d'un tube de prélèvement peut comprendre un tube flexible qui relie fluidiquement un tube de prélèvement agencé à distance de la tête de ponction et la portion arrière de l'aiguille de prélèvement ou directement un tube de prélèvement associé à des moyens d'arrimage/désarrimage du tube à l'arrière de l'aiguille comme décrit ultérieurement ou un organe de liaison entre un tube de prélèvement et un flexible porté directement par la portion arrière de l'aiguille.

Dans le cas où les tubes de prélèvement ne sont pas embarqués sur la tête de ponction, le dispositif de mise en liaison fluidique comprend par exemple un moyen de réception d'un flexible monté sur la portion arrière de l'aiguille de prélèvement. Ce moyen de réception peut par exemple prendre la forme d'une canule fixée à l'arrière de l'aiguille et destinée à venir s'emmancher dans un flexible relié à un tube de prélèvement agencé à distance de la machine de prélèvement sur laquelle la tête de ponction selon l'invention est montée. Cet emmanchement est par exemple réalisé par des moyens motorisés.

Une tête de ponction selon l'invention comprend également un porte-aiguille configuré pour recevoir une aiguille et des moyens de déplacement linéaire du porte-aiguille permettant ainsi, sur commande, d'armer la tête en vue d'insérer l'aiguille dans le membre du patient par l'activation de l'ensemble mécatronique.

L'insertion d'une aiguille dans le membre d'un patient s'effectue donc en deux étapes successives. Dans une première étape, la tête de ponction est armée et l'aiguille est mise en position de ponction par les moyens de déplacement du porte-aiguille. Dans une deuxième étape, l'ensemble mécatronique déplace la tête linéairement au niveau du point de ponction déterminée et selon la direction déterminée.

L'opération de retrait de l'aiguille, en situation normale, s'effectue aussi en deux étapes. Dans une première étape, la tête est déplacée selon la direction inverse de la direction de ponction jusqu'à ce qu'elle sorte du membre du patient. Dans une deuxième étape, une fois l'aiguille extraite du membre du patient, le dispositif électromécanique de retrait est activé pour désarmer la tête.

En cas d'urgence, le retrait de l'aiguille est réalisé en une seule étape qui consiste à activer le dispositif électromécanique de retrait pour assurer un retrait de l'aiguille. Ce retrait de l'aiguille est assuré par des moyens exclusivement mécaniques de telle sorte que ce retrait d'urgence puisse être réalisé y compris en cas d'absence de courant électrique.

Le dispositif électromécanique de désarmement de la tête et de retrait d'urgence présente donc une double fonction. En d'autres termes, les mêmes moyens sont utilisés à la fois pour le retrait de l'aiguille en situation normale et pour l'arrêt du prélèvement en cas d'urgence.

En outre et selon l'invention, ledit dispositif de mise en liaison fluidique est motorisé et comprend un dispositif de chargement et de distribution de tubes de prélèvement sanguin comprenant au moins un logement de réception d'un tube de prélèvement et au moins un actionneur d'arrimage/désarrimage de ce tube de prélèvement à l'arrière d'une aiguille montée sur ledit porte-aiguille, ledit actionneur d'arrimage/désarrimage étant configuré pour déplacer ledit tube de déplacement du logement de réception vers la portion arrière de ladite aiguille et inversement.

Ainsi, le tube de prélèvement est embarqué sur la tête et peut être déplacé de son logement de réception à l'arrière de l'aiguille par l'actionneur d'arrimage.

En d'autres termes, les tubes sont embarqués sur la tête et la mise en liaison fluidique est automatique et assurée par l'arrimage du tube de prélèvement à l'arrière du tube.

Avantageusement et selon l'invention, ledit dispositif de chargement et de distribution de tubes de prélèvement comprend un barillet comprenant une pluralité de logements de réception de tubes répartis autour d'un axe de rotation dudit barillet, ledit axe de rotation s'étendant parallèlement à l'axe de ladite aiguille de prélèvement, une fois l'aiguille montée sur ledit porte-aiguille, et écarté de cet axe d'une distance égale à la distance qui sépare ledit axe de rotation dudit barillet de chacun desdits logements de réception desdits tubes, de sorte que chaque tube puisse être aligné à ladite aiguille, par rotation dudit barillet, et arrimé à l'arrière de ladite aiguille, sous l'effet dudit actionneur d'arrimage formé d'un actionneur linéaire de déplacement du tube logé dans le logement de réception aligné à ladite aiguille.

Selon cette variante avantageuse, le barillet est monté sur le châssis de sorte que les logements de réception des tubes puissent, par rotation du barillet, s'étendre en regard de l'aiguille de prélèvement.

Une aiguille au sens de l'invention est de préférence formée d'une aiguille de veine biseautée destinée à rentrer dans la veine du membre du patient à ponctionner (qui forme alors la portion biseautée de ladite aiguille de prélèvement) et d'une aiguille perce-bouchon qui s'étend dans l'axe de l'aiguille biseautée et qui est destinée à s'étendre dans un tube de prélèvement pour y amener le sang prélevé (qui forme alors la portion arrière de ladite aiguille de prélèvement).

Ainsi, une tête de ponction selon cette variante de l'invention permet de remplir plusieurs tubes de prélèvement sans nécessiter un déplacement de la tête à la recherche d'un nouveau tube. En effet, après chaque remplissage d'un tube, le tube rempli peut être séparé de l'aiguille perce-bouchon et repositionné dans le barillet. Ce dernier peut alors pivoter d'un angle prédéterminé pour positionner un tube vide en face de l'aiguille, qui est arrimé à l'arrière de l'aguille perce-bouchon de manière à pouvoir recueillir un deuxième échantillon de sang prélevé, et ainsi de suite pour tous les tubes du barillet, sans nécessité de retirer l'aiguille de veine du membre du patient, et sans l'intervention d'un opérateur extérieur.

Avantageusement et selon cette variante, lesdits logements de réception des tubes de prélèvement ménagés à la périphérie dudit barillet de chargement et de distribution sont des anses de clipsage desdits tubes.

Selon cette variante, les tubes de prélèvement sont montés dans le barillet motorisé de chargement et de distribution de tubes de prélèvement sanguin par des anses de clipsage. Cela facilite le chargement des tubes et permet également de ne pas obstruer les tubes (à l'exception de la partie du tube enserrée dans l'anse de clipsage). Selon une variante avantageuse, les logements de réception sont régulièrement répartis autour de l'axe central de rotation du barillet.

Avantageusement et selon l'invention, le tête comprend en outre un pose-pansement motorisé comprenant une platine ventouse de maintien d'un pansement portée par un arbre creux pivotant par rapport audit châssis entre une position de chargement d'un pansement dans laquelle ladite ventouse peut venir en contact d'un distributeur de pansements extérieur à ladite tête pour pouvoir aspirer un pansement par la mise sous vide dudit arbre creux, à une position de pose du pansement, dans laquelle ledit pansement porté par ladite ventouse vient en contact appuyé avec la peau dudit membre du patient au niveau de la zone de ponction, lors du retrait de ladite aiguille dudit membre du patient pour pouvoir apposer ledit pansement sur ledit membre du patient par mise à la pression atmosphérique dudit arbre creux, libérant ainsi ledit pansement.

Une tête de ponction selon cette variante avantageuse de l'invention permet d'apposer automatiquement un pansement au niveau de la zone de ponction tout en réalisant un point de compression permettant d'éviter tout risque de saignement interne ou externe. Ce pose-pansement comprend une ventouse qui est configurée pour pouvoir aspirer un pansement fourni par un distributeur de pansements (extérieur à la tête et agencé au voisinage du patient à ponctionner), par la mise sous vide d'un tube creux portant la ventouse. Pour ce faire, la ventouse comprend de préférence une pluralité d'orifices arrangés sur sa surface supérieure en communication fluidique avec le tube creux, de sorte que la mise sous vide du tube creux permette, par différence de pression, d'aspirer un pansement mis en contact avec la surface supérieure de la ventouse, par déplacement de la ventouse dans ladite position de chargement. De préférence, le distributeur de pansements est chargé en un rouleau de pansements formé d'un substrat sur lequel sont apposés les pansements espacés les uns des autres d'une distance prédéterminée. Bien entendu, d'autres configurations sont possibles sans remettre en cause le principe de cette variante de réalisation. La ventouse vient en contact avec un pansement, aspire le pansement, et le pose-pansement est prêt à être utilisé. Le distributeur de pansements est de préférence motorisé pour qu'après aspiration d'un pansement, il tourne d'un angle prédéterminé pour présenter un pansement disponible pour une prochaine ponction. De préférence, les pansements sont non poreux et présentent une rigidité par rapport au substrat pour faciliter l'aspiration par la ventouse.

L'arbre creux du pose-pansement est monté pivotant par rapport audit châssis entre ladite position de chargement d'un pansement et ladite position de pose du pansement. Ainsi, le pose-pansement peut, dans la position de pose du pansement, exercer une pression prédéterminée sur le membre du patient en accentuant légèrement le pivotement de l'arbre creux. La position de pose du pansement est par exemple déterminée par la mesure d'un couple résistant au niveau de l'actionneur électrique assurant le pivotement de l'arbre creux.

La surface supérieure de la ventouse peut présenter selon une variante avantageuse de l'invention, un renfoncement permettant le passage de l'aiguille lors de la pose du pansement. Cette variante permet ainsi la concomitance des opérations de pose du pansement et de retrait de l'aiguille.

De préférence, la ventouse est pivotante par rapport au tube, ce qui lui permet de s'adapter à la courbure du membre du patient à ponctionner, indépendamment de la rotation et/ou de l'inclinaison de la tête de ponction.

Avantageusement et selon l'invention, ledit châssis comprend une partie fixe équipée des moyens de fixation amovible audit ensemble mécatronique et une partie mobile portant au moins ledit porte-aiguilles et ledit dispositif de mise en liaison fluidique, et en ce que ledit dispositif électromécanique de retrait d'urgence comprend un électro-aimant et un moyen de rappel s'étendant entre la partie fixe dudit châssis et ladite partie mobile dudit châssis, et configuré pour assurer le rappel de la partie mobile dudit châssis vers la partie fixe dudit châssis lorsque ledit électro-aimant n'est plus alimenté en courant.

Un dispositif électromécanique d'une tête selon cette variante forme ainsi un dispositif de sécurité qui permet un retrait immédiat de l'aiguille du bras du patient en cas d'urgence.

En fonctionnement normal, l'aiguille est retirée de la peau du patient à la fin de la ponction par l'activation des moyens de déplacement du porte-aiguille. Le dispositif électromécanique est ensuite activé pour désarmer la tête.

En cas d'urgence, le retrait de l'aiguille est assuré par un déplacement de la partie mobile du châssis par rapport à la partie fixe du châssis qui est déclenché par l'électro-aimant, ce qui provoque le désengagement complet de l'aiguille par l'activation du ressort de rappel.

Avantageusement et selon l'invention, la tête comprend en outre des moyens de détermination du niveau de remplissage du tube de prélèvement en liaison fluidique avec ladite aiguille de prélèvement.

Cette variante avantageuse permet de déterminer le niveau de remplissage des tubes, ce qui autorise notamment un suivi en temps réel de la ponction. Ces moyens de détermination du taux de remplissage peuvent par exemple être formés par des moyens optiques de détection de la présence de sang dans le tube de remplissage en liaison fluidique avec l'aiguille de prélèvement. Ces moyens peuvent également être formés de moyens de pesée du tube permettant de déterminer la quantité de sang présent dans le tube à partir d'une connaissance du poids à vide du tube. Ces moyens peuvent aussi être formés par des moyens de calcul du temps de remplissage permettant d'estimer la quantité de sang présent dans le tube à partir du temps de ponction et d'une information représentative du débit de prélèvement sanguin. Bien entendu, d'autres moyens peuvent être utilisés pour déterminer le niveau de remplissage du tube de prélèvement.

Cette variante permet également de détecter le remplissage des tubes et donc de piloter le changement d'un tube rempli par un tube vide à remplir dès que le niveau de remplissage atteint un niveau prédéterminé.

En particulier et avantageusement, les moyens de détermination du niveau de remplissage du tube de prélèvement sont configurés pour pouvoir déclencher un changement automatique du tube de prélèvement.

Par exemple, dans le cas où la tête est équipée d'un barillet de chargement et de distribution des tubes, la détection d'un certain niveau de remplissage du tube arrimé à l'aiguille entraine le déplacement du tube rempli dans son logement de réception, la rotation du barillet pour aligner un tube vide à l'arrière de l'aiguille, puis le déplacement de ce tube vide pour assurer l'arrimage du tube à l'arrière de l'aiguille. Cela permet de poursuivre le prélèvement sanguin avec un nouveau tube, sans aucune intervention extérieure. Le processus peut se reproduire pour l'ensemble des tubes prévus pour l'examen pratiqué.

En d'autres termes, selon cette variante, la détection d'un niveau de remplissage prédéterminé dans un tube de prélèvement pilote l'actionneur d'arrimage/désarrimage de ce tube de prélèvement à l'arrière d'une aiguille montée sur ledit porte-aiguille, et pilote le changement de tube (rotation du barillet dans le cas où le dispositif de chargement de tubes comprend un barillet).

Avantageusement et selon l'invention, la tête comprend en outre des moyens de détection de l'entrée de ladite aiguille montée sur ledit porte-aiguille dans une veine du patient à ponctionner.

Cette variante avantageuse permet de piloter le déplacement de l'aiguille dans le membre du patient à ponctionner et en particulier de déterminer le moment où l'aiguille est dans la veine à ponctionner.

Avantageusement et selon l'invention, la tête comprend en outre des capteurs agencés au voisinage dudit porte-aiguille et configurés pour détecter une présence anormale de sang dans ce voisinage.

Ces capteurs sont par exemple des capteurs résistifs, capacitifs ou inductifs configurés pour permettre de détecter la présence de sang au voisinage des zones qui ne devraient pas, en fonctionnement nominal, être affectées par du sang. Ces zones sont voisines du circuit fluidique de collecte du sang.

Avantageusement et selon l'invention, ledit porte-aiguille comprend des moyens de fixation par vissage, clipsage ou aimantation de ladite aiguille de prélèvement sur ledit porte-aiguilles.

Dans le cas de moyens de fixation par vissage, ils sont par exemple formés par un écrou double filetage, chaque filetage étant conformé au pas de vis d'un type d'aiguille disponible dans le commerce. Ainsi, la tête selon l'invention peut recevoir différents types d'aiguilles qui se distinguent l'un de l'autre notamment par la forme du filetage.

De préférence, ledit porte-aiguille comprend en outre un système de débrayage en rotation de ladite aiguille de manière à permettre une rotation de l'aiguille sur elle-même pour pouvoir orienter le biseau de ladite aiguille à l'opposé de la peau du membre du patient à ponctionner.

Cette caractéristique préférentielle permet d'orienter correctement le biseau pour faciliter l'insertion de l'aiguille dans la peau du membre du patient.

Avantageusement et selon l'invention, la tête comprend en outre des moyens de désinfection automatique de la zone de ponction.

Ces moyens de désinfection automatique comprennent par exemple un spray embarqué sur la tête, un système de pose d'un coton imbibé de produits désinfectants ou tous moyens équivalents.

L'invention concerne également une machine de prélèvement sanguin automatique ou semi-automatique d'un membre d'un patient comprenant un ensemble mécatronique, une unité de commande dudit ensemble mécatronique, un système de capture et de traitement d'images du membre du patient configuré pour déterminer une zone de ponction optimale, caractérisée en ce qu'elle comprend en outre une tête de ponction selon l'invention montée sur ledit ensemble mécatronique.

Une machine selon l'invention permet d'automatiser le prélèvement sanguin dans un membre d'un patient. Cette machine comprend, en plus de la tête de ponction selon l'invention, un ensemble mécatronique tel qu'un bras robotisé, une unité de commande permettant de commander la position du bras robotisé et un système de capture d'images et de traitement de ces images configuré pour permettre de définir un point de ponction optimal. Ce point de ponction optimal est ensuite fourni à l'unité de commande du bras robotisé et à l'unité de commande de la tête de ponction pour permettre d'insérer l'aiguille dans le bras du patient au niveau de la zone de ponction optimale déterminée par le système de traitement d'images. Ce système de traitement d'images est par exemple celui décrit dans la demande WO2015158978 au nom du demandeur.

Le système de traitement d'images peut également être directement embarqué sur la tête de ponction selon l'invention, par exemple porté par le châssis, de manière à pouvoir faire l'acquisition du membre du patient à ponctionner et déterminer la zone de ponction optimale. Cette variante permet à la tête d'embarquer toutes les fonctionnalités nécessaires à la ponction, la machine de prélèvement sanguin ayant alors pour principal rôle de commander les déplacements de l'ensemble mécatronique portant la tête de ponction.

Les avantages et effets décrits en lien avec la tête de ponction s'appliquent *mutatis mutandis* à la machine de prélèvement selon l'invention.

L'invention concerne également une tête de ponction et une machine de prélèvement sanguin automatique ou semi-automatique caractérisées en combinaison par tout ou partie des caractéristiques mentionnées ci-dessus ou ci-après.

### Liste des figures

D'autres buts, caractéristiques et avantages de l'invention apparaîtront à la lecture de la description suivante donnée à titre uniquement non limitatif et qui se réfère aux figures annexées dans lesquelles :
[Fig. 1] est une vue schématique en perspective avant d'une tête de ponction selon un mode de réalisation de l'invention,
[Fig. 2] est une vue schématique en perspective selon une autre orientation de la tête de ponction selon le mode de réalisation de la figure 1,
[Fig. 3] est une vue schématique en perspective selon une autre orientation de la tête de ponction du mode de réalisation des figures 1 et 2,
[Fig. 4] est une vue schématique de côté d'un porte-aiguille équipé d'une aiguille de ponction d'une tête de ponction selon un mode de réalisation de l'invention,
[Fig. 5] est une vue schématique d'une machine de prélèvement sanguin automatique selon un mode de réalisation de l'invention.

### Description détaillée d'un mode de réalisation de l'invention

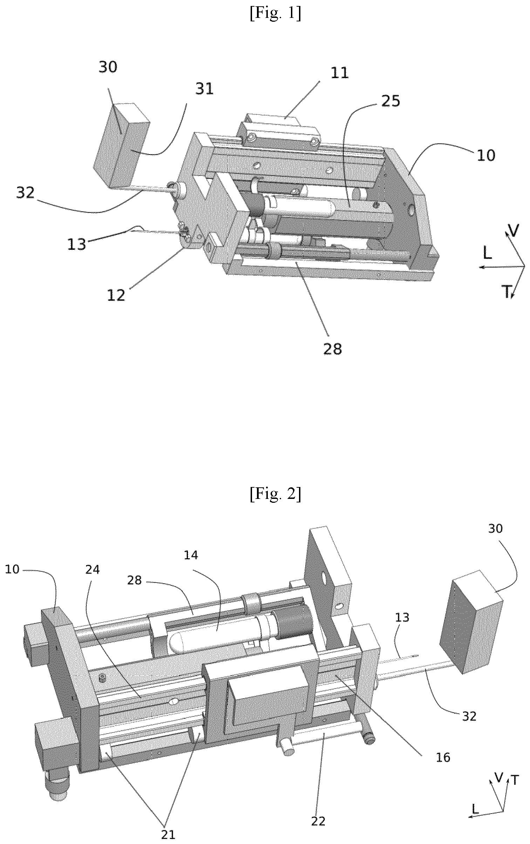

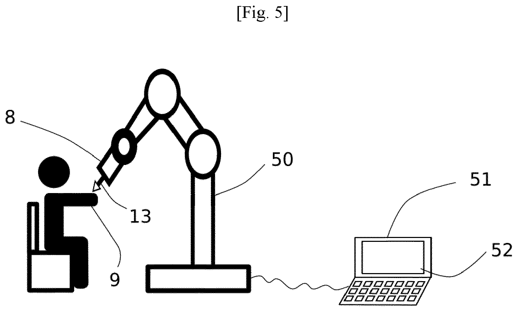

Sur les figures, les échelles et les proportions ne sont pas strictement respectées et ce, à des fins d'illustration et de clarté. Dans toute la description détaillée qui suit en référence aux figures, sauf indication contraire, chaque élément de la tête de ponction selon l'invention est décrit tel qu'il est lorsque la tête est montée sur un ensemble mécatronique tel qu'un bras robotisé d'une machine de prélèvement automatique. Le bras robotisé n'est en revanche pas représenté sur les figures 1 à 4 à des fins de clarté. Le bras est représenté schématiquement sur la figure 5.

En outre, les éléments identiques, similaires ou analogues sont désignés par les mêmes références dans toutes les figures.

Enfin, les termes longitudinal, transversal, vertical et leurs variantes sont utilisés à titre non limitatif en référence au trièdre L, T, V tel que représenté sur la figure 1 notamment. La direction longitudinale (référencée L sur les figures) correspond à la direction principale de la tête de ponction et coïncide notamment avec la direction le long de laquelle s'étend l'aiguille de prélèvement sanguin, lorsqu'elle est montée sur le porte-aiguille. La direction verticale (référencée V sur les figures) est la direction définie par la gravité et la direction transversale (référencée T sur les figures) est la direction perpendiculaire à la direction longitudinale et à la direction verticale.

La tête de ponction sanguine selon le mode de réalisation des figures comprend un châssis 10 équipé de moyens 11 de fixation amovible du châssis 10 à un bras robotisé d'une machine automatique de prélèvement sanguin, non représenté sur les figures 1 à 4 à des fins de clarté. Ces moyens 11 de fixation peuvent être de tous types et dépendent du bras robotisé sur lequel la tête de ponction est montée.

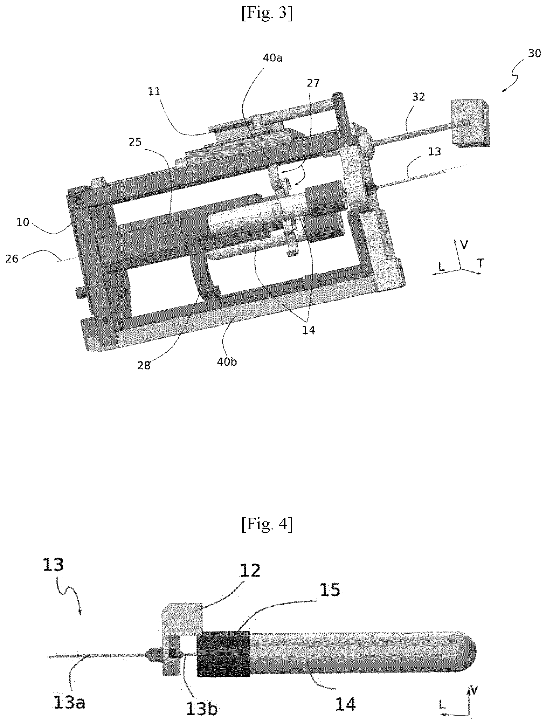

La tête de ponction comprend également un porte-aiguille 12 de prélèvement porté par le châssis 10 et configuré pour recevoir une aiguille 13, telle qu'une aiguille de prélèvement sanguin. Ce porte-aiguille 12 est représenté de manière plus détaillée sur la figure 4, qui illustre également un tube de prélèvement 14 arrimé à l'arrière de l'aiguille. L'aiguille de prélèvement 13 est formée d'une portion biseautée 13a destinée à rentrer dans la veine du membre du patient à ponctionner et d'une portion perce-bouchon 13b qui s'étend dans l'axe de la portion biseautée 13a et qui traverse un bouchon 15 pour s'étendre dans le tube 14 de prélèvement. Cette portion biseautée 13a et cette portion perce-bouchon 13b peuvent être formées d'une seule et même aiguille ou de deux aiguilles reliées l'une à l'autre.

Dans toute la suite, nous faisons référence à une aiguille biseautée et à une aiguille perce-bouchon, étant entendu qu'elles peuvent être réalisées par une seule et même aiguille comme indiqué précédemment. Les aiguilles 13a biseautée et 13b porte-bouchon sont en communication de fluide de sorte que le sang prélevé par l'aiguille 13a biseautée circule vers l'aiguille perce-bouchon 13b, puis vers le tube 14 de prélèvement arrimé à l'arrière de l'aiguille perce-bouchon.

L'aiguille de prélèvement 13 est montée sur le porte-aiguille 12 par vissage, clipsage ou aimantation.

Selon un mode de réalisation de l'invention, le porte-aiguille 12 comprend également des moyens de débrayage de l'aiguille 13 qui permettent de pivoter l'aiguille 13 sur son axe principal pour permettre d'orienter le biseau de l'aiguille à l'opposé de la peau du patient à ponctionner, ce qui facilite son insertion dans le membre du patient à ponctionner.

Ce débrayage est par exemple obtenu par un filetage du porte-aiguille monté pivotant autour de l'axe de l'aiguille et maintenu plaqué contre une platine de maintien par un ressort. La force de ce ressort, sa tension et la nature des matériaux utilisés sont configurées pour générer un frottement contrôlé, ce qui permet d'obtenir un couple de vissage suffisant pour assurer un blocage optimal en fin de vissage de l'aiguille, un entrainement en rotation du filetage permettant d'assurer le positionnement correct du biseau de l'aiguille et l'absence de mouvement de l'aiguille lors de l'insertion de l'aiguille.

Le porte-aiguille 12 est solidarisé au châssis 10 de la tête par tous moyens mécaniques possibles.

La tête de ponction comprend également des moyens de déplacement linéaire 16 du porte-aiguille 12 configurés pour permettre, une fois le porte-aiguille 12 équipé d'une aiguille 13 de prélèvement, d'armer la tête en vue de l'insertion de cette aiguille dans le membre d'un patient à ponctionner.

Ces moyens de déplacement linéaire du porte-aiguille sont de préférence formés du bras robotisé portant la tête de ponction à l'insertion de l'aiguille dans le membre du patient et du bras robotisé et d'un système de désarmement (ou de déclenchement) électromécanique lors du retrait de l'aiguille.

Ce système de déclenchement électromécanique est représenté schématiquement sur la figure 2. Il comprend notamment un électro-aimant 21 et un ressort de rappel 22 configuré pour assurer le rappel du porte-aiguille le long de glissières 24 lorsque ledit électro-aimant 21 n'est plus alimenté en courant.

En fonctionnement normal, une ventouse magnétique formant l'électro-aimant est alimentée en courant de sorte que le ressort de rappel 22 est maintenu étiré. Le système de déclenchement est conservé dans cette configuration pendant toute la ponction. Pour déclencher le système, en fin de ponction, en cas de retrait d'urgence ou en cas de coupure d'électricité, la ventouse magnétique libère la partie mobile de la tête qui est alors déplacée par la force du ressort 22 le long des glissières jusqu'à une butée d'amortissement.

Selon un mode de réalisation de l'invention non représenté sur les figures, le porte-aiguille 12 peut également comprendre un capteur de présence d'une aiguille adapté pour détecter la présence de l'aiguille sur le porte-aiguille. Un tel capteur peut par exemple être un capteur optique, un capteur mécanique, un capteur magnétique, ou tout moyen équivalent.

Selon un autre mode de réalisation non représenté sur les figures, le châssis comprend une partie fixe équipée des moyens de fixation amovible au bras robotisé, et une partie mobile portant le porte-aiguille, la partie mobile étant configurée pour pouvoir être déplacée, sur commande d'une unité de commande et d'actionneurs associés, par rapport à la partie fixe pour assurer l'insertion/retrait de l'aiguille portée par le porte-aiguille dans le membre du patient, ladite partie mobile formant ainsi lesdits moyens de déplacement linéaire dudit porte-aiguille.

Selon le mode de réalisation des figures, la tête de ponction comprend également un barillet 25 motorisé de chargement et de distribution de tubes 14 de prélèvement sanguin.

Ce barillet 25 comprend une pluralité de logements 27 de réception de tubes 14 répartis autour d'un axe de rotation 26 du barillet, formant un barillet étoilé. Chaque logement 27 de réception est formé d'une anse de clipsage élastique. Selon d'autres modes de réalisation, chaque logement de réception peut être formé par un double clipsage ce qui permet d'améliorer le maintien et assurer l'alignement des tubes.

L'axe de rotation 26 du barillet s'étend parallèlement à l'axe de l'aiguille de prélèvement 13, une fois l'aiguille montée sur le porte-aiguille 12, mais désaxé par rapport à l'axe de l'aiguille. Cet axe de rotation 26 est entrainé en rotation par un moteur électrique, non visible sur les figures. Ce moteur électrique est de préférence logé dans l'axe de rotation 26. Bien entendu, d'autres moyens électromécaniques peuvent être mis en oeuvre pour assurer cette rotation de l'axe de rotation 26 du barillet 25.

La rotation du barillet 25 autour de l'axe de rotation 26 permet de positionner, à tour de rôle, chaque tube de prélèvement 14 logé dans un logement de réception 27 dans l'axe de l'aiguille 13. Une fois le tube 14 de prélèvement dans l'axe de l'aiguille 13, le tube est déplacé, sous l'action d'un actionneur 28 linéaire bidirectionnel, vers l'arrière de l'aiguille 13 pour y être arrimé. Une fois le tube arrimé à l'arrière de l'aiguille 13, le prélèvement sanguin peut commencer. L'actionneur 28 linéaire étant bidirectionnel, il peut également assurer le retrait du tube de prélèvement, une fois ce dernier rempli, pour le remplacer par un autre tube du barillet 25.

La tête de ponction 8 selon le mode de réalisation des figures comprend également un pose-pansement 30 motorisé comprenant une platine ventouse 31 de maintien d'un pansement portée par un arbre creux 32 pivotant par rapport au châssis 10. Un tel actionneur bidirectionnel est par exemple un actionneur à pignon crémaillère.

Le pivotement de l'arbre creux 32 permet de déplacer la platine ventouse entre une position de chargement d'un pansement dans laquelle la ventouse peut venir en contact d'un distributeur de pansements extérieur à la tête pour pouvoir aspirer un pansement par la mise sous vide de l'arbre creux 32, à une position de pose du pansement, dans laquelle le pansement porté par la ventouse vient en contact avec la peau du membre du patient au niveau de la zone de ponction, lors du retrait de l'aiguille 13 du membre du patient pour pouvoir apposer le pansement sur le membre du patient par mise à la pression atmosphérique de l'arbre arbre creux 32, libérant ainsi le pansement.

Le pivotement de l'arbre creux 32 est assuré par exemple par un moteur électrique entrainant en rotation une rondelle d'engrenage engrenant une rondelle d'engrenage solidaire de l'arbre creux 32. Bien entendu, d'autres moyens électromécaniques peuvent être mis en oeuvre pour assurer le pivotement de l'arbre creux de la position d'aspiration du pansement à la position de pose du pansement sur le membre du patient.

Selon un mode de réalisation de l'invention non représenté sur les figures, le pose-pansement 30 peut également comprendre un capteur de détection d'un pansement. Un tel capteur peut par exemple être un capteur optique, un capteur mécanique, un capteur de dépression ou tout moyen équivalent.

La tête de ponction comprend également de préférence et tel qu'illustré sur la figure 3 des moyens de contrôle du remplissage 40a, 40b, de présence et de positionnement des tubes. Ces moyens de contrôle du remplissage 40a, 40b comprennent, selon le mode de réalisation des figures, des capteurs optiques configurés pour détecter la présence de sang dans les tubes de prélèvement 14 et le taux de remplissage des tubes. Il est ainsi possible de suivre en temps réel le déroulement du prélèvement sanguin. Ces capteurs permettent également de détecter la présence et le positionnement nominal du tube avant la ponction. Tous types de capteurs optiques peuvent être utilisés pour procéder à cette détection de sang dans le tube et le niveau de remplissage du tube. Selon le mode de réalisation des figures, un moyen 40a de remplissage est agencé du côté de l'émission des tubes, et un moyen 40b de remplissage est agencé du côté de la réception des tubes.

La tête de ponction selon le mode de réalisation des figures comprend également des capteurs résistifs agencés au voisinage du porte-aiguille 12 et du barillet 25 de chargement et de distribution des tubes et configurés pour détecter une présence anormale de sang dans ce voisinage.

La tête de ponction comprend également une unité de commande configurée pour recevoir les données et/ou mesures des différents capteurs et actionneurs de la tête permettant, en lien avec l'ensemble mécatronique, de piloter le fonctionnement et le déplacement de la tête de ponction.

L'invention concerne aussi une machine de prélèvement sanguin automatique illustrée très schématiquement sur la figure 5. Cette machine comprend, selon le mode de réalisation représenté, un bras robotisé 50, une unité de commande 51 du bras robotisé, un système de capture et de traitement d'images 52 du membre du patient configuré pour déterminer une zone de ponction optimale, et une tête de ponction 8 selon le mode de réalisation des figures 1 à 4. Le système de capture et de traitement d'images 52 comprend par exemple une caméra, non représentée sur les figures, adaptée pour faire l'acquisition du membre du patient 9 à ponctionner. Les images sont ensuite transmises à un module de traitement d'images configuré pour détecter une veine de ponction optimale. Le procédé d'identification de la veine optimale est par exemple celui décrit dans la demande WO2015158978 au nom du demandeur. Une fois la veine optimale détectée, l'unité de commande 52 transmet aux actionneurs du bras robotisé 50 et aux actionneurs de la tête 8 de ponction, les informations de déplacement du porte-aiguille de manière à permettre l'insertion de l'aiguille 13 dans le membre du patient 8 à prélever.

Préalablement à l'opération de prélèvement sanguin, la tête de ponction selon l'invention est chargée avec les tubes de prélèvement nécessaires à l'analyse spécifique du patient. Les tubes sont insérés dans le barillet rotatif, qui comprend de préférence un nombre de logements de réception de tubes, supérieur ou égal au nombre de tubes nécessaires à l'examen pratiqué sur le patient.

La tête est ensuite chargée avec un pansement. Comme expliqué précédemment, ce chargement du pansement peut être obtenu par le déplacement du bras creux 32 portant la platine ventouse 31 vers la position de chargement d'un pansement dans laquelle la ventouse peut venir en contact d'un distributeur de pansements.

Selon une autre variante, le distributeur de pansements peut être remplacé par un opérateur qui vient directement apposer le pansement sur la platine ventouse 31. De même, les opérations de chargement de tubes, de montage des aiguilles ou de désinfection peuvent être, selon une variante de réalisation, assurées par un opérateur.

Dès que le système de capture et de traitement d'images a défini le point de ponction optimal, la tête de ponction est mise en position de prélèvement par le bras robotisé. La tête avance vers le membre du patient à ponctionner et l'aiguille pénètre dans la peau selon un angle prédéterminé, soit par le module de traitement d'images, soit par un dispositif ou moyen annexe jusqu'à ce qu'elle atteigne la veine cible. Le premier tube de prélèvement correspondant à l'ordonnance est ensuite inséré sur l'aiguille perce-bouchon selon le mécanisme décrit précédemment.

Le remplissage du tube s'effectue et lorsqu'un niveau de remplissage prédéterminé est atteint (détecté par les moyens de contrôle de remplissage des tubes), le tube rempli est remplacé par un tube vide du barillet. Ce cycle d'opérations est répété jusqu'à complétion des prélèvements visés pour le patient considéré.

Une fois tous les prélèvements effectués, l'aiguille est retirée du membre du patient et le pansement présent sur la ventouse est posé sur le membre du patient au niveau du point de ponction.

## Revendications

1. Tête de ponction sanguine destinée à équiper un ensemble mécatronique tel qu'un bras robotisé, d'une machine de prélèvement sanguin automatique ou semi-automatique configuré pour permettre le déplacement de ladite tête au-dessus d'un membre d'un patient à ponctionner, ladite tête de ponction sanguine comprenant :
- un châssis (10) équipé de moyens de fixation (11) amovible du châssis audit ensemble mécatronique,
- un porte-aiguille (12) porté par ledit châssis (10) et adapté pour recevoir une aiguille (13) comprenant une portion biseautée (13a) destinée à percer la peau du membre du patient à ponctionner et une portion arrière (13b) destinée à permettre l'écoulement du sang prélevé vers un tube de prélèvement, **caractérisée en ce que** ladite tête de ponction sanguine comprend en outre :
- des moyens de déplacement linéaire (16) dudit porte-aiguille configurés pour permettre, une fois ce dernier équipé d'une aiguille, d'armer la tête de ponction en vue d'insérer, sur commande dudit ensemble mécatronique, cette aiguille (13) dans ledit membre dudit patient pour pouvoir effectuer un prélèvement sanguin,
- un dispositif de mise en liaison fluidique de ladite portion arrière de ladite aiguille (13) montée sur ledit porte-aiguilles (12) avec un tube de prélèvement destiné à recueillir le sang prélevé dans ledit membre du patient, ledit dispositif de mise en liaison fluidique étant motorisé et comprenant en outre un dispositif (25) de chargement et de distribution de tubes de prélèvement sanguin comprenant au moins un logement de réception (27) d'un tube de prélèvement (14) et au moins un actionneur d'arrimage (28) de ce tube de prélèvement à l'arrière d'une aiguille montée sur ledit porte-aiguilles, ledit actionneur d'arrimage (28) étant configuré pour déplacer ledit tube (14) de prélèvement du logement de réception vers la portion arrière de ladite aiguille et inversement,
- un dispositif électromécanique (21, 22) de désarmement de la tête et de retrait d'urgence de ladite aiguille (13) dudit membre du patient configuré pour permettre, sur commande, un retrait exclusivement mécanique de ladite aiguille dudit membre du patient.

2. Tête de ponction selon la revendication 1, **caractérisée en ce que** ledit dispositif (25) de chargement et de distribution de tubes de prélèvement comprend un barillet comprenant une pluralité de logements de réception de tubes répartis autour d'un axe de rotation (26) dudit barillet, ledit axe de rotation (26) s'étendant parallèlement à l'axe de ladite aiguille (13) de prélèvement, une fois l'aiguille montée sur ledit porte-aiguilles, et écarté de cet axe d'une distance égale à la distance qui sépare ledit axe de rotation dudit barillet de chacun desdits logements de réception desdits tubes, de sorte que chaque tube puisse être aligné à ladite aiguille, par rotation dudit barillet, et arrimé à l'arrière de ladite aiguille, sous l'effet dudit actionneur d'arrimage (28) formé d'un actionneur linéaire de déplacement du tube logé dans le logement de réception aligné à ladite aiguille.

3. Tête de ponction selon la revendication 2, **caractérisée en ce que** lesdits logements de réception des tubes de prélèvement ménagés à la périphérie dudit barillet de chargement et de distribution comprennent des anses de clipsage desdits tubes.

4. Tête de ponction selon l'une des revendications 1 à 3, **caractérisée en ce qu'**elle comprend en outre un pose-pansement (30) motorisé comprenant une platine ventouse (31) de maintien d'un pansement portée par un arbre creux (32) pivotant par rapport audit châssis entre une position de chargement d'un pansement dans laquelle ladite ventouse (31) peut venir en contact d'un distributeur de pansements extérieur à ladite tête pour pouvoir aspirer un pansement par la mise sous vide dudit arbre creux, à une position de pose du pansement, dans laquelle ledit pansement porté par ladite ventouse vient en contact appuyé avec la peau dudit membre du patient au niveau de la zone de ponction, lors du retrait de ladite aiguille dudit membre du patient pour pouvoir apposer ledit pansement sur ledit membre du patient par mise à la pression atmosphérique dudit arbre creux, libérant ainsi ledit pansement.

5. Tête de ponction selon la revendication 4, **caractérisée en ce que** ladite platine ventouse (31) du pose-pansement comprend un renfoncement permettant le passage de l'aiguille lors de la pose du pansement.

6. Tête de ponction selon l'une des revendications 4 ou 5, **caractérisée en ce que** ladite platine ventouse (31) est pivotante par rapport à l'arbre creux (12) de manière à pouvoir s'adapter à la courbure du membre du patient à ponctionner, indépendamment de la rotation et/ou de l'inclinaison de la tête de ponction.

7. Tête de ponction selon l'une des revendications 1 à 6, **caractérisée en ce que** ledit châssis (10) comprend une partie fixe équipée des moyens de fixation amovible audit ensemble mécatronique et une partie mobile portant au moins ledit porte-aiguilles et ledit dispositif de mise en liaison fluidique, et **en ce que** ledit dispositif électromécanique de désarmement et de retrait d'urgence comprend un électro-aimant et un moyen de rappel s'étendant entre la partie fixe dudit châssis et ladite partie mobile dudit châssis, et configuré pour assurer le rappel de la partie mobile dudit châssis vers la partie fixe dudit châssis lorsque ledit électro-aimant n'est plus alimenté en courant.

8. Tête de ponction selon l'une des revendications 1 à 7, **caractérisée en ce qu'**elle comprend en outre des moyens de détermination (40a, 40b) du niveau de remplissage du tube de prélèvement en liaison fluidique avec ladite aiguille de prélèvement.

9. Tête de ponction selon la revendication 8, **caractérisée en ce que** lesdits moyens de détermination du niveau de remplissage du tube de prélèvement sont configurés pour pouvoir déclencher un changement automatique du tube de prélèvement.

10. Tête de ponction selon l'une des revendications 1 à 9, **caractérisée en ce qu'**elle comprend en outre des moyens de détection de l'entrée de ladite aiguille montée sur ledit porte-aiguilles dans une veine du patient à ponctionner.

11. Tête de ponction selon l'une des revendications 1 à 10, **caractérisée en ce que** ledit porte-aiguilles (12) comprend des moyens de fixation par vissage, clipsage ou aimantation de ladite aiguille de ponction sur ledit porte-aiguilles.

12. Tête de ponction selon la revendication 11, **caractérisée en ce que** ledit porte-aiguilles (12) comprend en outre un système de débrayage en rotation de ladite aiguille de manière à permettre une rotation de l'aiguille sur elle-même pour pouvoir orienter le biseau de ladite aiguille à l'opposé de la peau du membre du patient à ponctionner.

13. Tête de ponction selon l'une des revendications 1 à 12, **caractérisée en ce qu'**elle comprend en outre des moyens de désinfection automatique de ladite zone de ponction.

14. Machine de ponction automatique ou semi-automatique d'un membre d'un patient comprenant un ensemble mécatronique (50), une unité de commande (52) dudit ensemble mécatronique, un système de capture et de traitement d'images du membre du patient configuré pour déterminer une zone de ponction optimale, **caractérisée en ce qu'**elle comprend en outre une tête de ponction (8) selon l'une des revendications 1 à 13, montée sur ledit ensemble mécatronique.

## Patentansprüche

1. Blutentnahmekopf zur Ausrüstung einer mechatronischen Einheit, wie z.B. eines Roboterarms, einer automatischen oder halbautomatischen Blutentnahmemaschine, die ausgestaltet ist, die Bewegung des Kopfes über eine Extremität eines Patienten, in die eingestochen werden soll, zu ermöglichen, wobei der Blutentnahmekopf umfasst:
- ein Gestell (10), das mit Mitteln zur lösbaren Befestigung (11) des Gestells an der mechatronischen Einheit ausgestattet ist,
- eine Nadelhalterung (12), die von dem Gestell (10) getragen wird und zur Aufnahme einer Nadel (13) geeignet ist, welche einen abgeschrägten Abschnitt (13a) zum Durchstechen der Haut der zu punktierenden Extremität des Patienten und einen hinteren Abschnitt (13b), aufweist, der den Abfluss des entnommenen Blutes in ein Entnahmeröhrchen ermöglicht, **dadurch gekennzeichnet, dass** der Blutentnahmekopf weiter umfasst:
- Mittel zur linearen Versetzung (16) der Nadelhalterung, die ausgestaltet sind, nach Ausrüstung mit einer Nadel, den Entnahmekopf damit zu koppeln, um, auf Befehl der mechatronischen Einheit, die Nadel (13) in die Extremität des Patienten zu insertieren, um eine Blutprobe zu entnehmen,
- eine Einrichtung zur fluiden Verbindung des hinteren Bereichs der an der Nadelhalterung (12) angebrachten Nadel (13) mit einem Entnahmeröhrchen zum Gewinnen des von den Extremitäten des Patienten genommenen Bluts, wobei die Einrichtung zur Herstellung der Fluidverbindung motorbetrieben ist und weiter eine Vorrichtung (25) zum Laden und Ausgeben von Blutentnahmeröhrchen umfasst, welche mindestens einen Aufnahmeraum (27) für ein Entnahmeröhrchen (14) und mindestens ein Koppelungsstellglied (28) zum Andocken des Entnahmeröhrchens am hinteren Teil einer auf der Nadelhalterung vorgesehenen Nadel umfasst, wobei das Koppelungsstellglied (28) ausgestaltet ist, das Entnahmeröhrchen (14) vom Aufnahmeraum zum hinteren Bereich der Nadel und umgekehrt zu bewegen,
- eine elektromechanische Vorrichtung (21, 22) zum Abkoppeln des Kopfes und zum Entfernen der Nadel (13) im Notfall aus den Extremitäten des Patienten, die ausgestaltet ist, auf Befehl ein ausschließlich mechanisches Entfernen der Nadel aus dem Extremitäten des Patienten zu ermöglichen.

2. Blutentnahmekopf nach Anspruch 1, **dadurch gekennzeichnet, dass** die Vorrichtung (25) zum Laden und Ausgeben von Entnahmeröhrchen eine Trommel mit mehreren Röhrchen-Aufnahmekammern umfasst, die um eine Drehachse (26) der Trommel verteilt sind, wobei die Drehachse (26) parallel zur Achse der Nadel (13) verläuft, sobald die Nadel auf der Nadelhalterung eingesetzt ist, und von dieser Achse in einer Entfernung beabstandet ist, die gleich der Entfernung ist, die die Drehachse der Trommel von dem Gehäuse, in der die Röhrchen aufgenommen sind, trennt, so dass jedes Röhrchen durch Drehung der Trommel mit der Nadel ausgerichtet ist, und am hinteren Bereich der Nadel mittels des Kopplungsstellglieds (28) gesichert ist, welches durch ein lineares Stellglied ausgestaltet ist, um das in der Aufnahmekammer befindliche, mit der Nadel ausgerichtete Röhrchen zu bewegen.

3. Blutentnahmekopf nach Anspruch 2, **dadurch gekennzeichnet, dass** die am Umfang der Lade- und Abgabetrommel ausgebildeten Kammern zur Aufnahme der Probenröhrchen Henkel zum Einclipsen der Röhrchen aufweisen.

4. Blutentnahmekopf nach einem der Ansprüche 1 bis 3, **dadurch gekennzeichnet, dass** er weiter eine motorisierte Wundverband-Halterung (30) umfasst, die einen Saugnapf (31) zum Halten eines Wundverbands aufweist, der von einer Hohlwelle (32) getragen wird, indem ein Unterdruck an die Hohlwelle angelegt wird, wobei die Hohlwelle relativ zu dem Gestell schwenkbar ist zwischen einer Position zum Aufnehmen eines Wundverbands, bei der der Saugnapf (31) mit einem Wundverband-Spender außerhalb des Kopfes in Kontakt treten kann, um einen Wundverband anzusaugen, und einer Position zum Aufbringen des Wundverbands, bei der der von dem Saugnapf getragene Wundverband mit der Haut der Extremitäten des Patienten im Bereich der Einstichstelle in Druck-Kontakt kommt, während die Nadel aus den Extremitäten des Patienten entfernt wird, um den Wundverband auf den Extremitäten des Patienten durch unter Druck setzen des Hohlschaftes aufbringen zu können, wodurch der Wundverband freigegeben wird.

5. Blutentnahmekopf nach Anspruch 4, **dadurch gekennzeichnet, dass** der Saugnapf (31) der Wundverband-Halterung eine Vertiefung umfasst, die den Durchgang der Nadel beim Anlegen des Wundverbands ermöglicht.

6. Blutentnahmekopf nach einem der Ansprüche 4 oder 5, **dadurch gekennzeichnet, dass** der Saugnapf (31) relativ zur Hohlwelle (12) schwenkbar ist, so dass sie sich unabhängig von der Drehung und/oder Neigung des Blutentnahmekopf an die Krümmung der Extremität des zu punktierenden Patienten anpassen kann,

7. Blutentnahmekopf nach einem der Ansprüche 1 bis 6, **dadurch gekennzeichnet, dass** das Gestell (10) aufweist, ein fixiertes Teil, das mit Mitteln zur lösbaren Befestigung an der mechatronischen Baugruppe ausgestattet ist, und ein bewegliches Teil umfasst, das mindestens eine Nadelhalterung und die Vorrichtung zur Herstellung einer Fluidverbindung aufweist, und dass die elektromechanische Vorrichtung zur Entkoppelung und zum Herausziehen im Notfall einen Elektromagneten und ein Rückstellmittel umfasst, das sich zwischen dem fixierten Teil des Gestells und dem beweglichen Teil des Gestells erstreckt und so ausgestaltet ist, dass es das bewegliche Teil des Gestells zum fixierten Teil des Gestells zurückstellt, wobei das bewegliche Teil des Gestells in Richtung des fixierten Teils des Gestells zurückgestellt wird, wenn der Elektromagnet nicht mehr mit Strom versorgt wird.

8. Blutentnahmekopf nach einem der Ansprüche 1 bis 7, **dadurch gekennzeichnet, dass** er weiter Mittel zur Bestimmung (40a, 40b) des Füllstands des Entnahmerohrs in Fluidverbindung mit der Entnahmenadel umfasst.

9. Blutentnahmekopf nach Anspruch 8, **dadurch gekennzeichnet, dass** die Mittel zur Bestimmung des Füllstands des Entnahmerohrs so ausgestaltet sind, dass sie einen automatischen Wechsel des Entnahmerohrs auslösen können.

10. Blutentnahmekopf nach einem der Ansprüche 1 bis 9, **dadurch gekennzeichnet, dass** er weiter Mittel zum Erfassen des Eintritts der auf der Nadelhalterung montierten Nadel in eine Ader des zu punktierenden Patienten umfasst.

11. Blutentnahmekopf nach einem der Ansprüche 1 bis 10, **dadurch gekennzeichnet, dass** die Nadelhalterung (12) Mittel zur Befestigung der Punktionernadel auf der Nadelhalterung durch Schrauben, Clipsen oder Magnetisieren umfasst.

12. Blutentnahmekopf nach Anspruch 11, **dadurch gekennzeichnet, dass** die Nadelhalterung (12) weiter ein System zum Auskuppeln der Drehung der Nadel umfasst, so dass eine Drehung der Nadel um die eigene Achse ermöglicht wird, um die Schräge der Nadel von der Haut der zu punktierenden Extremität des Patienten weg zu richten.

13. Blutentnahmekopf nach einem der Ansprüche 1 bis 12, **dadurch gekennzeichnet, dass** er weiter Mittel zum automatischen Desinfizieren des Einstichbereichs umfasst.

14. Maschine zur automatischen oder halbautomatischen Punktion einer Extremität eines Patienten, umfassend, eine mechatronische Baugruppe (50), eine Steuereinheit (52) der mechatronischen Baugruppe, ein System zur Erfassung und Verarbeitung von Bildern der Extremität des Patienten, das ausgestaltet ist, einen optimalen Punktierbereich zu bestimmen, **dadurch gekennzeichnet, dass** sie weiter einen Blutentnahmekopf (8) nach einem der Ansprüche 1 bis 13 umfasst, der an der mechatronischen Baugruppe angebracht ist.

## Claims

1. Blood puncture head intended to equip a mechatronic assembly such as a robotic arm, of an automatic or semi-automatic blood sampling machine configured to allow movement of said head above a limb of a patient to be punctured, said blood puncture head comprising:
- a frame (10) equipped with means for removably fixing (11) the frame to said mechatronic assembly,
- a needle holder (12) carried by said frame (10) and adapted to receive a needle (13) comprising a beveled portion (13a) intended to pierce the skin of the patient's limb to be punctured and a rear portion (13b) intended to allow the flow of collected blood to a collection tube,
**characterized in that** said blood puncture head further comprises:
- linear displacement means (16) of said needle holder configured to allow, once the latter is equipped with a needle, to arm the puncture head with a view to inserting, on command from said mechatronic assembly, this needle (13) in said limb of said patient in order to be able to take a blood sample,
- a device for fluidically connecting said rear portion of said needle (13) mounted on said needle holder (12) with a collection tube intended to collect the blood taken from said patient's limb, said fluidically connecting device being motorized and further comprising a device (25) for loading and distributing blood collection tubes comprising at least one housing (27) for receiving a collection tube (14) and at least one securing actuator (28) of this collection tube to the rear of a needle mounted on said needle holder, said securing actuator (28) being configured to move said collection tube (14) from the receiving housing to the rear portion of said needle and vice versa,
- an electromechanical device (21, 22) for disarming the head and emergency removal of said needle (13) from said patient's limb configured to allow, on command, an exclusively mechanical removal of said needle from said patient's limb.

2. Puncture head according to claim 1, **characterized in that** said device (25) for loading and distributing sampling tubes comprises a barrel comprising a plurality of housings for receiving tubes distributed around an axis of rotation (26) of said barrel, said axis of rotation (26) extending parallel to the axis of said needle (13), once the needle is mounted on said needle holder, and separated from this axis by a distance equal to the distance which separates said axis of rotation of said barrel from each of said housings for receiving said tubes, so that each tube can be aligned with said needle, by rotation of said barrel, and secured at the rear of said needle, under the effect of said securing actuator (28) formed by a linear actuator for moving the tube housed in the receiving housing aligned with said needle.

3. Puncture head according to claim 2, **characterized in that** the said housings for receiving the sampling tubes formed at the periphery of the said loading and dispensing barrel comprise loops for clipping the said tubes.

4. Puncture head according to one of claims 1 to 3, **characterized in that** it further comprises a motorized dressing holder (30) comprising a suction cup plate (31) for holding a dressing carried by a hollow shaft (32 ) pivoting relative to said frame between a position for loading a dressing in which said suction cup (31) can come into contact with a dressing dispenser outside said head in order to be able to suck up a dressing by placing the said hollow shaft under vacuum, in a position for placing the dressing, in which said dressing carried by said suction cup comes into pressed contact with the skin of said patient's limb at the level of the puncture zone, during withdrawal of said needle from said patient's limb in order to be able to affix said dressing on said patient's limb by venting said hollow shaft, thereby releasing said dressing.

5. Puncture head according to claim 4, **characterized in that** the said suction cup plate (31) of the dressing holder comprises a recess allowing the passage of the needle during the fitting of the dressing.

6. Puncture head according to one of claims 4 or 5, **characterized in that** the said suction plate (31) is pivotable with respect to the hollow shaft (12) so as to be able to adapt to the curvature of the patient's limb at puncture, independent of the rotation and/or inclination of the puncture head.

7. Puncture head according to one of claims 1 to 6, **characterized in that** the said frame (10) comprises a fixed part fitted with means for removable fixing to the said mechatronic assembly and a mobile part carrying at least the said needle holder and the said fluidic connection, and **in that** said electromechanical device for disarming and emergency withdrawal comprises an electromagnet and a return means extending between the fixed part of said frame and said movable part of said frame, and configured to ensure the return of the movable part of said frame to the fixed part of said frame when said electromagnet is no longer supplied with current.

8. Puncture head according to one of claims 1 to 7, **characterized in that** it further comprises means (40a, 40b) for determining the filling level of the collection tube in fluidic connection with the said sampling needle.

9. Puncture head according to Claim 8, **characterized in that** the said means for determining the filling level of the collection tube are configured to be able to trigger an automatic change of the sampling tube.

10. Puncture head according to one of claims 1 to 9, **characterized in that** it further comprises means for detecting the entry of said needle mounted on said needle holder into a vein of the patient to be punctured.

11. Puncture head according to one of claims 1 to 10, **characterized in that** the said needle-holder (12) comprises means for fixing by screwing, clipping or magnetization of the said puncture needle on the said needle-holder.

12. Puncture head according to claim 11, **characterized in that** the said needle holder (12) further comprises a system for disengaging the rotation of the said needle so as to allow rotation of the needle on itself in order to be able to orient the bevel said needle away from the skin of the patient's limb to be punctured.

13. Puncture head according to one of claims 1 to 12, **characterized in that** it also comprises means for the automatic disinfection of the said puncture zone.

14. Machine for automatic or semi-automatic puncture of a patient's limb comprising a mechatronic assembly (50), a control unit (52) of said mechatronic assembly, a system for capturing and processing images of the patient's limb configured to determine an optimal puncture zone, **characterized in that** it further comprises a puncture head (8) according to one of claims 1 to 13, mounted on said mechatronic assembly.
